# EUROPÄISCHE PATENTANMELDUNG

(11) **EP 1 980 293 A1**
(43) Veröffentlichungstag der Anmeldung: **15.10.2008**
(21) Anmeldenummer: 08102745.0
(22) Anmeldetag: 19.03.2008
(51) Int. Cl.: A61Q 5/12, A61Q 5/02, A61Q 5/00, A61K 8/81

(54) **Zubereitung zur Pflege des Haares, enthaltend ein ampholytisches Copolymer**

(30) Priorität: 11.04.2007 DE 102007017307
(71) Anmelder: Beiersdorf AG, 20245 Hamburg (DE)
(72) Erfinder: Argembeaux, Horst, 21465 Wentorf (DE); Kohut, Michaela, 20255 Hamburg (DE); Ruppert, Stefan, 20259 Hamburg (DE); Streicher, Harald, 20146 Hamburg (DE)
(74) Vertreter: Porath, Stefan

(57) **Zusammenfassung**

Kosmetische Zubereitung enthaltend ein von DIQUAT und Acrylsäure abgeleitetes Copolymer und Parfum.

## Beschreibung

Neben der Reinigung ist die Pflege des Haares von essenzieller Bedeutung um das Haar in seinem gesunden Zustand zu erhalten oder dieses in einen solchen Zustand zu versetzen. Solches Haar zeichnet sich durch einen angenehmen Griff, natürlichen Glanz, Geschmeidigkeit und somit guter Frisierbarkeit und Festigkeit und somit gutem Frisurensitz aus. Gepflegtes Haar zeichnet sich darüber hinaus durch eine gewisse Frische und einen angenehmen Duft aus.

Zur Reinigung von Haaren werden üblicherweise Shampoos benutzt. Moderne Shampoos bieten dem Haar neben der Reinigung noch ein gewisses Maß an Pflege. Derartige Shampoos sind meist mittelviskose Flüssigkeiten, die als transparente oder trübe bis perglänzende Produkte in Flaschen oder Tuben angeboten werden. Nach dem Waschprozess werden die Shampoos aus dem Haar ausgespült.

Produkte die ausschließlich der Pflege des Haares dienen werden allgemein als Conditioner bezeichnet. Diese können nach einer mehr oder weniger langen Verweilzeit auf dem Haar ausgespült werden (Rinse-off Produkte, z. B. Spülungen, Haar-Kuren) oder sie Verbleiben nach der Anwendung auf dem Haar (Leave-on Produkte). Die Produkte können verschiedene Konsistenzen haben, so dass sie ganz unterschiedlich appliziert werden können. Es können Emulsionen oder Gele sein oder dünnflüssige Lösungen, die z. B. über Sprühapplikationen aufgebracht werden, oder Schäume, die z. B. durch geeignete Druckgaspackungen oder spezielle Schaumpumpen bei der Applikation erzeugt werden. Cremige, trübe und klar transparente Produkte sind im Markt zu finden.

Je nach Verwendungszweck findet man ganz unterschiedliche Wirkstoffe oder Kombinationen von Wirkstoffen in Shampoos oder Conditionern. Manche die eher dem Schutz des Haares dienen, wie Antioxidantien oder UV-Filter, andere die das Haar geschmeidig machen wie z. B. kationische Tenside oder Lipide. Eine immer größere Bedeutung bekommen polymere Wirkstoffe, die je nach Art, Molgewicht und Ladung ganz unterschiedliche Eigenschaften haben.

So gibt es Polymere, die die rheologischen Eigenschaften des Produktes beeinflussen, andere ziehen auf die Haaroberfläche auf und geben dem Haar je nach Polymereigenschaft Geschmeidigkeit, Glanz oder Halt. Es wird auch von Polymeren berichtet, die synergistisch die Wirkung von Haarkonditionierhilsmitteln wie z. B Silikonölen verbessern.

So beschreiben die Patentschriften EP 0917454, EP 0821579, EP 1004291, EP 1137397, EP 1140007 bereits Kombinationen von Silikonen mit kationischen Polymeren in Shampooformulierungen mit einer ausgezeichneten konditionierenden Wirkung.

Haarbehandlungsmittel der beschriebenen Art erfüllen ihre Grundfunktionen sehr gut, das behandelte Haar ist sauber und gepflegt, die Frisur sitzt. Ein Mangel solcher Haarbehandlungsmittel ist jedoch die Dauer dieses gepflegten Zustandes. Umwelteinflüsse, insbesondere Expositionen durch Rauch und üble Gerüche beeinträchtigen den Pflegezustand des Haares, es mangelt schnell an Frische und riecht "verbraucht".

Um Haarpflegemittel in ihrer Attraktivität für den Verbraucher zu steigern, wird schönes Aussehen eine angenehme Sensorik und ein angenehmer Duft bei der Entwicklung solcher Produkte berücksichtigt. Zur Erzielung eines angenehmen Duftes wird in der Regel das Produkt mit einem Duftingredient versetzt. Solche Ingredients gehören meist zur Gruppe der etherischen Öle und können einzeln, meist aber als speziell abgestimmte Gemische, den Parfums, den Produkten zugesetzt werden. Parfums verleihen den Produkten einen angenehmen Duft, der am Produkt selbst und bei der Benutzung des Produktes wahrgenommen wird. Unmittelbar nach der Benutzung kann dieser Duft deutlich abgeschwächt und zum Teil verfälscht auch noch am Haar wahrgenommen werden, nach kurzer Zeit ist dieser aber verflogen.

Überraschend wurde nun festgestellt, dass der Einsatz eines ampholytischen Copolymers in kosmetischen Zubereitungen, insbesondere Haarbehandlungsmitteln den Dufteindruck eines Parfums am Haar dahingehend beeinflusst, dass es diesen unverfälscht und lang anhaltend wieder gibt. Dem Haar wird somit eine sauberkeitsvermittelnde Frische und ein angenehmer Duft über einen längeren Zeitraum verliehen.

Erfindungsgemäße ampholytische Copolymere der beschriebenen Art leiten sich ab von Acrylsäure und/oder Methacrylsäure und Verbindungen der Formel

**H₂C=CR¹-Z-[CH₂]ₙ-N⁺R²R³-[A-N⁺R²R³]ₘ-B-N+R⁴R⁵R⁶ (m+2) X⁻**

R¹ stellt dabei H, Methyl oder Ethyl dar,
R², R³, R⁴, R⁵, R⁶ stellt dabei ein oder mehrere lineare oder verzweigte C₁ bis C₆, besonders bevorzugt C₁ bis C₄-Alkyl, Hydroxyalky- oder Aminoalkyl dar,
m stellt dabei 0 bis 10, besonders bevorzugt 2 bis 4 dar,
Z stellt dabei -(C=O)-O-, -(C=O)-NH- oder-O- dar,
A stellt dabei C1 bis C6, bevorzugt C2 bis C4-Alkylen dar,
B stellt eine lineare oder verzweigte C2 bis C12, besonders bevorzugt C3 bis C6 Polymethylenkette, optional unterbrochen mit einem oder mehreren Heteroatomen oder Heterogruppen wie O oder NH und optional mit einer oder mehreren Hydroxy- oder Amino-Gruppen, besonders bevorzugt Hydroxygruppen substituiert,
X- bedeutet ein oder mehrere gleiche oder verschiedene Gegenionen.

Besonders bevorzugt wird als Comonomer DIQUAT eingesetzt, um zu erfindungsgemäßen Polymeren zu gelangen. Dies ist durch folgende Formel gekennzeichnet:

**CH₃-(C=CH₂)-(C=O)-NH-(CH₂)₃-N⁺(CH₃)₂-CH₂-CHOH-CH₂-N⁺CH₃)₂ 2X⁻**

wobei X- Chlorid- oder Methylsulfationen bedeutet.

Das Gewichtsmittel des Molekulargewichts solcher Copolymere beträgt 10000 bis 10000000, besonders bevorzugt 500000 bis 2000000 g/mol

Ganz besonders bevorzugt sind die beiden Polymere A und B, die beide ein Gewichtsmittel des Molekulargewichts von etwa 1000000 haben:
Polymer A: Copolymer aus 33 % DIQUAT-Einheiten und 67 % Acrylsäureeinheiten
Polymer B: Copolymer aus 50 % DIQUAT-Einheiten und 50 % Acrylsäureeinheiten

Daher umfasst die Erfindung eine kosmetische Zubereitung enthaltend ein von DIQUAT und Acrylsäure abgeleitetes Copolymer und Parfum. Besonders bevorzugt ist es, wenn das Copolymer 25 bis 60 % molare Anteile an DIQUAT und 40 bis 75 % molare Anteile an Acrylsäure enthält. Ganz besonders bevorzugt ist es, wenn das Copolymer 30 bis 35 % molare Anteile an DIQUAT und 63 bis 70 % molare Anteile an Acrylsäure enthält oder wenn das Copolymer 45 bis 55 % molare Anteile an DIQUAT und 45 bis 55 % molare Anteile an Acrylsäure enthält. Ganz außergewöhnlich besonders bevorzugt ist es, wenn sich das Polymer im wesentlichen, also abgesehen von Verunreinigungen, nur aus DIQUAT und Acrylsäure aufbaut. Dabei ist es bevorzugt, wenn zusätzlich schäumende Tenside und/oder kationische Tenside enthalten sind. Dabei ist es bevorzugt, wenn zusätzlich kationische Polymere enthalten sind. Dabei ist es bevorzugt, wenn zusätzlich zyklische Silikone enthalten sind. Weiterhin bevorzugt ist es, wenn als Parfumbestandteile Aldehyde enthalten sind.

Die Erfindung umfasst auch die Verwendung von oben beschriebenen Copolymeren in kosmetischen Zubereitungen zur Verringerung der Verdampfung von Parfüm.

Erfindungsgemäße kosmetische Zubereitungen können darüber hin aus die üblichen Hilfs- und Zusatzstoffe enthalten.

Folgende Beispiele beschreiben die Erfindung:

### Shampoos

### 1) Conditioner-Shampoo

| | **1** | **2** | **3** | **4** | **5** | **6** | **7** |
|---|---|---|---|---|---|---|---|
| Natrium Laurylethersulfat | 7 | 7 | 8 | 9 | 9 | 10 | 12 |
| Cocamidopropyl Betain | - | 3 | 3.5 | 3.5 | 4 | 4 | 3 |
| Di-Natrium PEG-5 Laurylcitrat Sulfosuccinat | - | - | 2 | 3 | 3 | - | - |
| Decyl Glucoside | - | 2 | 3 | - | - | - | - |
| Natrium Cocoamphoacetat | 3 | - | - | - | - | - | - |
| Polymer A | - | 0.2 | - | 0.3 | 0.2 | - | 0.5 |
| Polymer B | 0.1 | - | 0,8 | - | - | 0.2 | - |
| Dimethicon | 1,0 | 2,0 | 1,5 | 0,8 | - | 1,0 | - |
| PEG-200 Hydrogenated Glyceryl Palmate | - | - | 0,2 | - | - | 1,0 | - |
| PEG-40 hydrogeniertes Rizinusöl | 0.2 | 0.3 | 0.4 | 0,5 | 0.6 | 0,5 | 0.5 |
| PEG-3 Distearat | 1.5 | - | 0.75 | 1.5 | 1.0 | - | 0.5 |
| Glycol Distearat | - | 4.0 | - | - | - | - | - |
| PEG-7 Glycerylcocoat | 0.5 | 1 | 2 | - | - | - | - |
| Natrium Salicylat | 0.2 | 0.3 | 0.4 | 0.4 | 0.4 | - | - |
| Natrium Benzoat | 0.5 | 0.4 | 0.3 | 0.2 | 0.4 | - | 0.5 |
| Parfüm | 0,3 | 0,5 | 0,3 | 0,3 | 0,4 | 0,7 | 0,3 |
| Benzophenon-4 | 0,2 | 0,25 | - | 0,3 | - | - | - |
| Oryzanol | - | - | - | 0,5 | - | - | 0,05 |
| Piroctone Olamine | - | - | - | - | - | 0.1 | 0.2 |
| Niacinamid | - | 0.01 | - | - | - | - | - |
| Panthenol | - | - | 0.01 | - | - | - | 0.01 |
| Jojobaöl | - | - | - | - | 0,1 | - | - |
| Natronlauge | q.s. | q.s. | q.s. | q.s. | q.s. | q.s. | - |
| Citronensäure | q.s. | q.s. | q.s. | q.s. | q.s. | q.s. | q.s. |
| Wasser | ad | ad | ad | ad | ad | ad | ad |
| | 100 | 100 | 100 | 100 | 100 | 100 | 100 |

### 2) Conditioner-Shampoo mit Perlglanz (und Trübungsmittel)

| | **1** | **2** | **3** | **4** | **5** | **6** |
|---|---|---|---|---|---|---|
| Natrium Laurylethersulfat | 9 | 9 | 9 | 9 | 9 | 9 |
| Cocamidopropyl Betain | 4 | 3 | 4 | 3 | 4 | 3 |
| Dinatrium PEG-5 Laurylcitrat Sulfosuccinat | 3 | 3 | 3 | 2 | 3 | 4 |
| PEG-200 Hydrogenated Glyceryl Palmate | - | - | 0.1 | - | 0,3 | 0,4 |
| Polymer A | - | - | 0.2 | 0.1 | - | 0,4 |
| Polymer B | 0.3 | 0.1 | - | - | 0.2 | - |
| Dimethicone | - | 1,0 | 0,5 | 0,4 | - | - |
| PEG-3 Distearat | 1.5 | 3 | 4 | 2 | 2,5 | 0,75 |
| Glycol Distearat | - | - | - | - | 0.5 | 0,5 |
| PEG-40 hydriertes Rizinusöl | 0.2 | 0.3 | 0.4 | 0.5 | 0.2 | 0,3 |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 3) Klares Conditioning-Shampoo

| | **1** | **2** | **3** | **4** | **5** | **6** |
|---|---|---|---|---|---|---|
| Natrium Laurylethersulfat | 10 | 9 | 3.5 | 3.5 | 0.5 | 8 |
| Natrium Myrethsulfat | - | - | 3.5 | 3.5 | 3.0 | 2 |
| Cocamidopropyl Betain | 4 | 4.5 | 3 | - | - | 3 |
| Natrium Cocoamphoacetat | - | - | - | 2.5 | - | 3 |
| Dinatrium PEG-5 Laurylcitrat Sulfosuccinat | - | - | - | - | 2.5 | 3 |
| Decylglucosid | - | - | - | 2 | 4.5 | - |
| PEG-200 Hydrogenated Glyce-ryl Palmate | 0.1 | - | - | 0.4 | 0.5 | - |
| Polymer A | 0,2 | - | - | 0,8 | 0,4 | - |
| Polymer B | - | 0,1 | 0,3 | - | - | 1,0 |
| Dimethicone | 1,0 | 1,5 | 0,5 | - | - | - |
| Hydrolysiertes Seidenprotein | - | 0,1 | 0,2 | 0,3 | 0.3 | - |
| PEG-40 hydriertes Rizinusöl | 0.1 | 0.2 | 0.3 | 0.1 | 0.2 | 0,4 |
| Natrium Salicylat | - | - | 0.4 | - | | 0,2 |
| Natrium Benzoat | 0.5 | 0.5 | 0.4 | 0.4 | 0.4 | 0,4 |
| Benzophenon-4 | - | - | 0.1 | 0,2 | 0,3 | 0,4 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 4) Mildes Baby Shampoo

| | **1** | **2** | **3** | **4** | **5** | **6** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyceryl Palmate | - | - | - | 0.2 | 0.3 | 0,4 |
| Polymer A | 0.3 | - | - | - | 0,4 | 0,5 |
| Polymer B | - | 0.1 | 0.2 | 0.3 | - | - |
| Dimethicon | 0,5 | 0,8 | 1,0 | - | - | - |
| PEG-3 Distearat | - | - | 0,5 | 2 | - | - |
| Glycol Distearat | - | - | - | - | 0.5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 5) Antischuppen Shampoo/Mildes Kopfhaut Shampoo

| | **1** | **2** | **3** | **4** | **5** | **6** |
|---|---|---|---|---|---|---|
| Natrium Laurylethersulfat | 9 | 9 | 9 | 9 | 10 | - |
| Natrium Myristylethersulfat | - | - | - | - | - | 6 |
| Cocamidopropyl Betain | 4 | 4 | 3 | 3 | 4 | 5 |
| Dinatrium PEG-5 Laurylcitrat Sulfosuccinat | 3 | 3 | 3 | 3 | - | - |
| Natrium Cocoamphoacetat | - | - | - | - | - | 2.5 |
| Decylglucosid | - | - | - | - | - | 2.5 |
| PEG-200 Hydrogenated Glyceryl Palmate | - | - | 0.1 | - | 0.4 | 3 |
| Polymer A | 0.3 | - | 0,2 | - | 0.1 | - |
| Polymer B | - | 0.1 | - | 0.3 | - | 0.5 |
| Dimethicone | 0,5 | 1,0 | 1,5 | - | - | 1,0 |
| Climbazol | 0.5 | 0.5 | - | 0.5 | 1.0 | - |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| PEG-3 Distearat | 1.5 | 3 | 0,75 | 0,5 | - | - |
| Glycol Distearat | - | - | - | - | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

### Beispiel Haarkuren:

| | **1** | **2** | **3** | **4** | **5** | **6** |
|---|---|---|---|---|---|---|
| Hydroxypropylmethylcellulose | 0,5% | 0,5% | 0,5% | 0,3% | 0,4% | 0,5% |
| Cetrimoniumbromid | 1,0 | - | 0,8 | - | 0,5 | 0,7 |
| Behentrimoniumchlorid | - | 0,7 | 0,3 | - | - | - |
| Distearoylethyl Hydroxyethylmonium Methosulfate | - | - | - | 1,2 | - | 0,7 |
| Palmitamidopropyltrimonium Chlorid | - | - | - | - | 0,5 | - |
| Glycerin | 3,0 | 3,0 | 3,0 | 2,5 | 2,0 | - |
| Cetearylalkoho | 2,5 | 2,5 | 2,5 | 2,5 | 2,0 | 2,5 |
| Glycerylstearat | 2,0 | 2,0 | 2,0 | 2,0 | 2,0 | 1,8 |
| Polyquaternium-10 | 0,1 | - | - | - | 0,1 | - |
| Guar Hydroxypropyl Trimonium Chlorid | - | 0,2 | - | 0,1 | - | - |
| Polymer A | - | - | 1,0 | - | 0,5 | 0,4 |
| Polymer B | 0,3 | 0,2 | - | 0,8 | - | - |
| Parfüm | 0,5 | 0,4 | 0,5 | 0,3 | 0,4 | 0,4 |
| Konservierungsmittel, pH-Einstellung und Lösungsvermittler | q.s. | q.s. | q.s. | q.s. | q.s. | q.s. |
| Wasser | ad 100 | ad 100 | ad 100 | ad 100 | ad 100 | ad 100 |

### Beispiel Haarspülungen:

| | 1 | 2 | 3 | 4 | 5 | 6 |
|---|---|---|---|---|---|---|
| Cetrimoniumchlorid | 1,0 | 0,5 | 0,5 | - | 0,5 | 0,5 |
| Distearoylethyl Hydroxyethylmonium Methosulfate | - | - | - | 1,0 | - | 0,5 |
| Palmitamidopropyltrimonium Chlorid | - | - | - | - | 0,8 | - |
| Behentrimoniumchlorid | - | 0,2% | 0,3 | - | - | - |
| Cetearylalkohol | 3,0 | 2,5 | 2,8 | 3,0 | 2,6 | 2,8 |
| Glycerin | 3,0 | 3,0 | 3,0 | 2,8 | 2,5 | 2,0 |
| Hydroxyethylcellulose | 0,2 | 0,2 | 0,2 | 0,2 | 0,2 | 0,2 |
| Polyquaternium-10 | 0,1 | - | - | - | - | 0,1 |
| Guar Hydroxypropyl Trimonium Chlorid | - | 0,2 | - | - | - | - |
| Polymer A | - | - | 0,4 | - | 1,0 | 0,4 |
| Polymer B | 0,5 | 0,4 | | 0,3 | | |
| Parfüm | 0,5 | 0,4 | 0,5 | 0,3 | 0,4 | 0,4 |
| Konservierungsmittel, pH-Einstellung und Lösungsvermittler | q.s. | q.s. | q.s. | q.s. | q.s. | q.s. |
| Wasser | ad 100 | ad 100 | ad 100 | ad 100 | ad 100 | ad 100 |

### Beispiel Spray-Conditioner:

| | 1 | 2 | 3 | 4 | 5 | 6 | 7 |
|---|---|---|---|---|---|---|---|
| Cetrimoniumchlorid | 0,2 | 0,1 | 0,8 | 0,5 | - | 0,2 | - |
| Behentrimoniumchlorid | - | 0,2 | 0,3 | - | - | - | - |
| Distearoylethyl Hydroxyethylmonium Methosulfate | - | - | - | - | 0,2 | - | 0,2 |
| Palmitamidopropyltrimonium Chlorid | - | - | - | - | - | 0,1 | 0,1 |
| Benzophenone-4 | 0,05 | 0,03 | - | - | - | - | 0,03 |
| PVP/VA Copolymer | - | 0,7 | - | - | - | 0,2 | - |
| Polyquaternium-10 | 0,1 | - | - | - | 0,1 | - | - |
| Polyquaternium-4 | 0,2 | - | - | - | - | 0,2 | 0,2 |
| Propylene Glycol | - | - | - | 3,0 | 2,0 | - | 2,0 |
| Polyquaternium-11 | - | - | 0,2 | - | - | - | - |
| Panthenol | 0,1 | - | 0,2 | 0,1 | - | 0,2 | - |
| Glyceryl lsostearate | - | - | - | 0,4 | - | - | - |
| Isoceteth-20 | - | - | - | 0,8 | - | - | - |
| Dicaprylyl Carbonate | - | - | - | 0,5 | - | - | - |
| Polymer A | - | 0,4 | - | 0,2 | 0,5 | - | 0,3 |
| Polymer B | 0,2 | - | 0,3 | - | - | 0,5 | - |
| Parfüm | 0,3 | 0,5 | 0,5 | 0,4 | 0,3 | 0,3 | 0,4 |
| Konservierungsmittel, pH-Einstellung und Lösungsvermittler | q.s. | q.s. | q.s. | q.s. | q.s. | q.s. | q.s. |
| Wasser | ad | ad | ad | ad | ad | ad | ad |
| | 100 | 100 | 100 | 100 | 100 | 100 | 100 |

### Beispiel Leave-on Conditioner:

| | 1 | 2 | 3 | 4 | 5 | 6 | 7 |
|---|---|---|---|---|---|---|---|
| Cetylalcohol | 1,5 | 1,8 | 2,0 | - | 1,0 | 1,7 | 2,0 |
| Cetrimoniumchlorid | 0,3 | 0,1 | 0,5 | 0,5 | - | 0,2 | - |
| Behentrimoniumchlorid | - | 0,2 | - | - | - | - | 0,2 |
| Distearoylethyl Hydroxyethylmonium Methosulfate | - | - | - | - | 0,5 | - | 0,2 |
| Palmitamidopropyltrimonium Chlorid | - | - | - | - | - | 0,2 | - |
| Benzophenone-4 | 0,05 | 0,03 | - | 0,1 | - | 0,1 | - |
| PVP/VA Copolymer | 0,4 | - | - | - | 0,3 | - | - |
| Polyquaternium-37 | - | - | - | 1,0 | - | - | - |
| Polyquaternium-4 | - | - | - | 0,2 | - | 0,1 | - |
| Polyquaternium-10 | - | - | 0,5 | - | 0,3 | - | 0,2 |
| Panthenol | 0,1 | - | 0,2 | 0,1 | - | - | 0,2 |
| Hydroxyethylcellulose | - | - | - | 0,3 | - | - | 0,2 |
| Acrylates/C10-30 Alkyl Acrylates Cross-polymer | 0,5 | 0,3 | 0,2 | - | - | - | - |
| C12-13 Alkyl Lactate | 2,0 | 1,0 | 1,5 | 1,0 | 1,5 | 1,0 | 1,0 |
| Laureth-4 | - | - | - | 0,5 | - | - | - |
| Aluminium Starch Octenylsuccinate | - | - | - | 1,0 | - | - | - |
| Dicaprylyl Carbonate | - | - | - | 1,0 | - | - | - |
| Polymer A | 0,2 | - | - | 0,1 | - | 0,3 | 0,2 |
| Polymer B | - | 0,2 | 0,3 | - | 0,5 | - | - |
| Parfüm | 0,5 | 0,4 | 0,3 | 0,4 | 0,5 | 0,4 | 0,4 |
| Konservierungsmittel, pH-Einstellung und Lösungsvermittler | q.s. | q.s. | q.s. | q.s. | q.s | q.s | q.s |
| Wasser | ad | ad | ad | ad | ad | ad | ad |
| | 100 | 100 | 100 | 100 | 100 | 100 | 100 |

### Beispiel Haarwässer:

| | 1 | 2 | 3 | 4 |
|---|---|---|---|---|
| Ethanol | 30,0 | 50,0 | - | - |
| Isopropanol | - | - | 40,0 | 30,0 |
| Panthenol | 0,2 | 0,1 | 0,2 | 0,2 |
| Menthol | 0,1 | - | 0,05 | 0,05 |
| Tocopheryl Acetate | 0,2 | 0,2 | - | 0,1 |
| C12-13 Alkyl Lactate | 0,2 | 0,1 | 0,2 | - |
| Polymer A | 0,1 | - | 0,2 | - |
| Polymer B | - | 0,1 | - | 0,1 |
| Parfüm | 0,3 | 0,4 | 0,3 | 0,3 |
| Konservierungsmittel, pH-Einstellung und Lösungsvermittler | q.s. | q.s. | q.s. | q.s. |
| Wasser | ad 100 | ad 100 | ad 100 | ad 100 |

## Patentansprüche

1. Kosmetische Zubereitung enthaltend ein von DIQUAT und Acrylsäure abgeleitetes Copolymer und Parfum.

2. Zubereitung nach Patentanspruch 1 **dadurch gekennzeichnet, dass** das Copolymer 25 bis 60 % molare Anteile an DIQUAT und 40 bis 75 % molare Anteile an Acrylsäure enthält.

3. Zubereitung nach einem der vorangehenden Patentansprüche **dadurch gekennzeichnet, dass** das Copolymer 30 bis 35% molare Anteile an DIQUAT und 63 bis 70% molare Anteile an Acrylsäure enthalten.
3 a) Zubereitung nach einem der vorangehenden Patentansprüche **dadurch gekennzeichnet, dass** das Copolymer 45 bis 55 % molare Anteile an DIQUAT und 45 bis 55 % molare Anteile an Acrylsäure enthalten.

4. Zubereitung nach einem der vorangehenden Patentansprüche **dadurch gekennzeichnet, dass** sich das Polymer im wesentlichen nur aus DIQUAT und Acrylsäure aufbaut. (gemeint ist abgesehen von Verunreinigungen)

5. Zubereitung nach einem der vorangehenden Patentansprüche **dadurch gekennzeichnet, dass** zusätzlich schäumende Tenside und/oder kationische Tenside enthalten sind.

6. Zubereitung nach einem der vorangehenden Patentansprüche **dadurch gekennzeichnet, dass** zusätzlich kationische Polymere enthalten sind.

7. Zubereitung nach einem der vorangehenden Patentansprüche **dadurch gekennzeichnet, dass** zusätzlich zyklische Silikone enthalten sind.

8. Zubereitung nach einem der vorangehenden Patentansprüche **dadurch gekennzeichnet, dass** als Parfumbestandteile Aldehyde enthalten sind.

9. Verwendung von Copolymeren nach einem der Patentansprüche 1 bis 4 in kosmetischen Zubereitungen zur Verringerung der Verdampfung von Parfüm.
